# EUROPEAN PATENT SPECIFICATION

(11) **EP 3 815 715 B1**
(45) Date of publication and mention of the grant of the patent: **11.09.2024**
(21) Application number: 19825285.0
(22) Date of filing: 28.06.2019
(51) Int. Cl.: C12N 15/115, A61K 9/08, A61K 48/00, A61K 31/7105, A61K 47/26, A61P 9/00, A61P 19/00, A61P 19/02, A61P 19/10, A61P 25/04, A61P 27/02, A61P 29/00

(54) **APTAMER PREPARATION**
APTAMERPRÄPARAT
PRÉPARATION D'APTAMÈRES

(30) Priority: 29.06.2018 JP 2018124390
(43) Date of publication of application: 05.05.2021
(73) Proprietor: RIBOMIC INC., Minato-ku Tokyo 108-0071 (JP)
(72) Inventor: NAKAMURA, Yoshikazu, Tokyo 108-0071 (JP); AKITA, Kazumasa, Tokyo 108-0071 (JP); ALI, Yusuf, Tokyo 108-0071 (JP)
(74) Representative: J A Kemp LLP
(86) International application number: PCT/JP2019/025766
(87) International publication number: WO 2020/004607

(56) References cited:
- EP-A1- 3 124 611
- WO-A1-2009/118108
- WO-A1-2015/147017
- CN-A- 101 317 820
- US-A1- 2011 053 795
- NAKAMURA YOSHIKAZU ED - SALGADO ANTONIO ET AL: "Aptamers as therapeutic middle molecules", BIOCHIMIE, vol. 145, 16 October 2017 (2017-10-16), pages 22 - 33, XP085338366, ISSN: 0300-9084, DOI: 10.1016/J.BIOCHI.2017.10.006
- PATEL, K. A. ET AL.: "Challenges with osmolytes as inhibitors of protein aggregation: Can nucleic acid aptamers provide an answer?", INT J BIOL MACROMOL., vol. 100, 2017, pages 75 - 88, XP085031809, ISSN: 0141-8130, DOI: 10.1016/j.ijbiomac.2016.05.014

## Description

### [Technical Field]

The present invention relates to an aptamer preparation, particularly an aptamer preparation containing an aptamer for a basic fibroblast growth factor (FGF2) as an active ingredient.

### [Background Art]

In recent years, applications of RNA aptamers to medicaments, diagnostic reagents, and test reagents have been drawing attention, and some RNA aptamers have already been in the stage of clinical study or practical use. The world's first RNA aptamer drug, Macugen (registered trade mark) (general name: pegaptanib sodium), was approved as a therapeutic drug for age-related macular degeneration in December 2004 in the US, and in July 2008 in Japan. Macugen (registered trade mark) contains an aptamer against VEGF as the living body. The aptamer is composed of a synthetic oligonucleotide consisting of 28 nucleic acid molecules, and a polyethylene glycol (PEG) derivative is bound to the 5'-terminal thereof.

The dosage form of Macugen (registered trade mark) is a prefilled syringe injection, and it is injected into the vitreous body. It is a clear aqueous injection that is colorless to slightly colored, and contains sodium hydrogen phosphate, sodium dihydrogen phosphate, an isotonic agent, and a pH adjuster. It has been shown that this preparation formulation is stable for 6 months in an accelerated test at 25±2°C and for 36 months in a long-term storage test at 5±3°C (non-patent document 1).

On the other hand, at the time of filing of the present application, there was no pharmaceutical product other than Macugen (registered trade mark) that contained an aptamer as the active ingredient, and it is not known at all what preparation formulation is appropriate for aptamer preparations.

The Applicant is developing a pharmaceutical product containing an aptamer for FGF2 as an active ingredient and applicable to age-related macular degeneration, achondroplasia, and cancer pain (patent documents 1 and 2). In the process of developing an FGF2 aptamer preparation, the present inventors obtained results that a preparation formulation considered to be almost the same as Macugen (registered trade mark) and a preparation formulation containing phosphate buffered saline (PBS) as a medium cannot maintain the activity of FGF2 aptamer. Patent document 3 discloses an aptamer for FGF2 and use thereof. Patent document 4 discloses a method of long term storage of substrate-coupled beads. Non-patent document 2 reports aptamers as possible middle-molecular weight drugs.

### [Document List]

### [Patent documents]

patent document 1: WO 2011/099576
patent document 2: WO 2015/147017
patent document 3: EP 3 124 611 A1
patent document 4: WO 2009/118108 A1

### [non-patent document]

non-patent document 1: Pharmaceutical product interview form, age-related macular degeneration therapeutic agent Macugen (registered trade mark) intravitreal injection kit 0.3 mg, March 2015 (revised 5th edition)
non-patent document 2: Nakamura, Y. (Biochemie 145(2018) 22-23)

### [Summary of Invention]

### [Technical Problem]

Therefore, the present invention aims to provide a preparation formulation capable of stably maintaining the activity of an aptamer, particularly an aptamer for FGF2, for a long term, thereby providing a pharmaceutical preparation containing an aptamer, particularly an FGF2 aptamer, as an active ingredient.

### [Solution to Problem]

The present inventors have conducted intensive studies in an attempt to achieve the above-mentioned purpose and found optimal preparation conditions for FGF2 aptamer, which resulted in the completion of the present invention.

The invention is set out in the appended claims.

### [Advantageous Effects of Invention]

According to the present invention, an easily handleable aptamer preparation can be provided because an aptamer for FGF2 or a salt thereof which is the active ingredient can be stably stored for a long term in the form of an aqueous liquid.

### [Description of Embodiments]

The present invention provides a pharmaceutical preparation containing an aptamer for FGF2 or a salt thereof as an active ingredient, wherein the aptamer or a salt thereof is stably maintained for a long term (hereinafter to be also referred to as "the aptamer preparation of the present invention"). As used herein, "stable for a long term" means that the proportion of a monomer aptamer after storage of the preparation enclosed in a glass bottle at 4°C for 3 months is not less than 70%. The proportion of the monomer aptamer is a value obtained by separating and detecting monomers and multimers by size-exclusion chromatography under the following conditions, and calculating (peak area of monomers)/(total peak area of monomers and multimers)×100(%).
apparatus: ACQUITY UPLC H-Class Bio manufactured by Waters
detector: TUV detector manufactured by Waters
column: ACQUITY UPLC BEH200 SEC column manufactured by Waters
sample concentration: 0.2 mg/mL
injection volume: 5 µL
eluent: 10% acetonitrile/PBS
flow rate: 0.3 mL/min
column temperature: 25°C

The aptamer preparation of the present invention contains an aptamer for FGF2 or a salt thereof as the active ingredient, and a non-electrolyte osmoregulator.

An aptamer refers to a nucleic acid molecule having a binding activity for a particular target molecule. The aptamer can inhibit the activity of a particular target molecule by binding to the target molecule. The aptamer to be the active ingredient of the aptamer preparation of the present invention is an aptamer having a binding activity to FGF2. In a preferred embodiment, the aptamer is an aptamer that can bind to FGF2 and inhibit the binding between FGF2 and FGF receptor. That is, the aptamer has an inhibitory activity against FGF2.

The aptamer used in the present invention is an aptamer that binds to FGF2, and further preferably an aptamer that can bind to FGF2 and inhibit the binding between FGF2 and FGF receptor. Whether or not the aptamer used in the present invention inhibits the binding between FGF2 and FGF receptor can be evaluated by, for example, a test using the surface plasmon resonance method such as Example 1 and the like.

While the aptamer for FGF2 is not particularly limited, for example, the aptamer described in WO 2015/147017, specifically, an aptamer containing a nucleotide sequence represented by the following formula (1) (wherein uracil is optionally thymine):

N¹GGAN²ACUAGGGCN³UUAAN⁴GUN⁵ACCAGUGUN⁶ (1)

and is the following (a) or (b):
(a) an aptamer wherein, in the nucleotides contained in the aptamer,
   (i) the 2'-position of the ribose of each pyrimidine nucleotide is a fluorine atom,
   (ii) the 2'-position of the ribose of each purine nucleotide is a hydroxy group;
(b) an aptamer wherein, in the nucleotides contained in the aptamer,
   (i) the 2'-position of the ribose of each pyrimidine nucleotide is independently unsubstituted, or substituted by an atom or group selected from the group consisting of a hydrogen atom, a methoxy group and a fluorine atom,
   (ii) the 2'-position of the ribose of each purine nucleotide is independently unsubstituted, or substituted by an atom or group selected from the group consisting of a hydrogen atom, a methoxy group and a fluorine atom.

In the above-mentioned formula (1), N¹ and N⁶ are each independently any 0 to several bases, and N², N³, N⁴ and N⁵ are independently any one base. In the present specification, "base" means any of adenine (A), guanine (G), cytosine (C), uracil (U) or thymine (T) constituting a nucleic acid.

While the base number of N¹ is not particularly limited as long as an aptamer containing a nucleotide sequence represented by the formula (1) binds to FGF2, it may be, for example, 0 - about 10, 0 - 9, 0 - 8, 0 - 7, 0 - 6, 0 - 5, 0 - 4, 0 - 3, 0 - 2 and the like, preferably 0 - 2.

Similarly, while the base number of N⁶ is not particularly limited, it may be, for example, 0 - about 10, 0 - 9, 0 - 8, 0 - 7, 0 - 6, 0 - 5, 0 - 4, 0 - 3 and the like, preferably 0 - 10, 3 - 9, or 5 - 8.

In a preferred embodiment, in the above-mentioned formula (1),
N¹ is G, GG, AG, C or gap,
N² is A or U,
N³ is G, C or A,
N⁴ is G, C or U,
N⁵ is G or U, and
N⁶ is UUCN⁶¹ or AGUCN⁶² wherein N⁶¹ and N⁶² are each independently any 0 to several bases. Here, N¹ is a "gap" means that N¹ is absent in the formula (1), namely, N¹ is 0 base.

While the base number of N⁶¹ is not particularly limited, it may be, for example, 0 - about 10, 0 - 7, 0 - 6, 0 - 5, 0 - 4 and the like, preferably 0 - 5, 1 - 5, or 2 - 4.

While the base number of N⁶² is also not particularly limited, it may be, for example, 0 - about 10, 0 - 7, 0 - 5, 0 - 4, 0 - 3 and the like, preferably 0 - 5, 0 - 4, or 0 - 3.

In another preferred embodiment, in the above-mentioned formula (1),
N¹ is G, GG, AG or gap,
N² is A or U,
N³ is G or A,
N⁴ is C or U,
N⁵ is G or U,
N⁶ is UUCN⁶¹ or AGUCN⁶² wherein N⁶¹ and N⁶² are as defined above.

In a preferred embodiment, the aptamer used in the present invention contains a nucleotide sequence represented by the following formula (2) or (3):

GGGAAACUAGGGCGUUAACGUGACCAGUGUUUCN⁶¹ (2)

N¹GGAUACUAGGGCAUUAAUGUUACCAGUGUAGUCN⁶² (3)

wherein N¹, N⁶¹ and N⁶² are as defined above, more preferably, a nucleotide sequence represented by the formula (3).

In a preferred embodiment, the aptamer used in the present invention contains a nucleotide sequence shown by any of SEQ ID NOs: 1 - 12. The nucleotide sequences shown in SEQ ID NOs: 1 - 12 are given below (wherein uracil is optionally thymine) (hereinafter A, G, C and U show that the base of nucleotide is adenine, guanine, cytosine or uracil, respectively):
SEQ ID NO: 1:
   GGGAUACUAGGGCAUUAAUGUUACCAGUGUAGUCUCGA
SEQ ID NO: 2:
   GGGAAACUAGGGCGUUAACGUGACCAGUGUUUCUCGA
SEQ ID NO: 3:
   GGGAUACUAGGGCAUUAAUGUUACCAGUGUAGUCCC
SEQ ID NO: 4:
   GGAUACUAGGGCAUUAAUGUUACCAGUGUAGUCC
SEQ ID NO: 5:
   GGGGAUACUAGGGCAUUAAUGUUACCAGUGUAGUCCCC
SEQ ID NO: 6:
   AGGGAUACUAGGGCAUUAAUGUUACCAGUGUAGUCCC
SEQ ID NO: 7:
   GGGAAACUAGGGCGUUAACGUGACCAGUGUUUCCC
SEQ ID NO: 8:
   CGGAUACUAGGGCAUUAAUGUUACCAGUGUAGUCCG
SEQ ID NO: 9:
   CCGAUACUAGGGCAUUAAUGUUACCAGUGUAGUCGG
SEQ ID NO: 10:
   GGGAUACUAGGGCGUUAACGUUACCAGUGUAGUCCC
SEQ ID NO: 11:
   GGGAUACUAGGGCCUUAAGGUUACCAGUGUAGUCCC
SEQ ID NO: 12:
   GGGAUACUAGGGCAUUUAUGUUACCAGUGUAGUCCC

In one preferred embodiment, the aptamer used in the present invention contains a nucleotide sequence shown in SEQ ID NO: 1, 3, 4, 5, 6, 8, 9, 10 or 12, more preferably, SEQ ID NO: 3, 8, 9, 10 or 12.

In another preferred embodiment, the aptamer used in the present invention contains a nucleotide sequence shown in SEQ ID NO: 2 or 7 (encompassed in the above-mentioned formula (2)).

In still another preferred embodiment, the aptamer used in the present invention contains a nucleotide sequence shown in SEQ ID NO: 1, 3, 4, 5, 6 or 8 (encompassed in the above-mentioned formula (3)).

In one embodiment, the aptamer used in the present invention may contain, in any of the above-mentioned nucleotide sequences, a nucleotide sequence wherein 1 or several nucleotides are substituted, deleted, inserted or added, as long as the aptamer still binds to FGF2, and may be
(a) an aptamer wherein, in the nucleotides contained in the aptamer,
   (i) the 2'-position of the ribose of each pyrimidine nucleotide is a fluorine atom,
   (ii) the 2'-position of the ribose of each purine nucleotide is a hydroxy group,; or
(b) an aptamer wherein, in the nucleotides contained in the aptamer,
   (i) the 2'-position of the ribose of each pyrimidine nucleotide is independently unsubstituted, or substituted by an atom or group selected from the group consisting of a hydrogen atom, a methoxy group and a fluorine atom,
   (ii) the 2'-position of the ribose of each purine nucleotide is independently unsubstituted, or substituted by an atom or group selected from the group consisting of a hydrogen atom, a methoxy group and a fluorine atom.

As used herein, the number of the above-mentioned nucleotides substituted, deleted, inserted or added is not particularly limited as long as the aptamer still binds to FGF2 even after the substitution, deletion, insertion or addition. It can be, for example, 1-about 10, preferably 1-6, more preferably 1-5, further preferably 1-4, further preferably 1-3, most preferably 1 or 2. While the site of the nucleotide to be substituted, deleted, inserted or added is not particularly limited as long as the aptamer still binds to FGF2 even after the substitution, deletion, insertion or addition, at the sites specified to be one kind of nucleotide in the above-mentioned formula (1), (2) and (3) (namely, A, G, C or U), nucleotides are substituted, deleted, inserted or added at 1 - 3, preferably 1 or 2, more preferably 1, site. On the other hand, when plural kinds of nucleotides may be present in the formulas (1), (2) and (3) (namely, N¹, N², N³, N⁴, N⁵, N⁶, N⁶¹ or N⁶²), more number of nucleotides (e.g., 1 - about 10, preferably 1 - 6, more preferably 1 - 5, further preferably 1 - 4) may be substituted, deleted, inserted or added. For example, when the nucleotide sequence shown in SEQ ID NO: 3 is the original sequence, SEQ ID NO: 1 is SEQ ID NO: 3 in which the 3'-terminal CC is substituted with UCGA, SEQ ID NO: 4 is SEQ ID NO: 3 in which one nucleotide is deleted from each of the both terminals, SEQ ID NO: 5 is SEQ ID NO: 3 in which G is added to the 5'-terminal and C is added to the 3'-terminal, SEQ ID NO: 6 is SEQ ID NO: 3 in which A is added to the 5'-terminal, SEQ ID NO: 8 is SEQ ID NO: 3 in which the 5'-terminal G is substituted with C, and the 3'-terminal C is substituted with G, SEQ ID NO: 9 is SEQ ID NO: 3 in which the 5'-terminal GG is substituted with CC, and the 3'-terminal CC is substituted with GG, SEQ ID NO: 10 is SEQ ID NO: 3 in which the 14th A is substituted with G, and the 19th U is substituted with C, and SEQ ID NO: 12 is SEQ ID NO: 3 in which the 17th A is substituted with U.

The length of the aptamer used in the present invention is not particularly limited, and can usually be about 10 to about 200 nucleotides and can be, for example, not less than about 20 nucleotides (e.g., not less than 25 nucleotides, not less than 30 nucleotides, not less than 31 nucleotides, not less than 32 nucleotides, not less than 33 nucleotides), preferably not less than 25 nucleotides, more preferably not less than 30 nucleotides, further preferably not less than 33 nucleotides. In addition, it can be, for example, not more than about 100 nucleotides, generally not more than about 80 nucleotides, preferably not more than about 70 nucleotides, more preferably not more than about 60 nucleotides, further preferably not more than about 50 nucleotides, further preferably not more than about 45 nucleotides (e.g., not more than 44 nucleotides, not more than 43 nucleotides, not more than 42 nucleotides, not more than 41 nucleotides, not more than 40 nucleotides). When the total number of nucleotides is smaller, chemical synthesis and mass-production will be easier, and there is a major advantage in terms of cost. It is also thought that chemical modification is easy, stability in the body is high, and toxicity is low.

Therefore, the length of the aptamer used in the present invention may be generally about 10 - about 200 nucleotides, preferably 20 - 80 nucleotides, more preferably 25 - 60 nucleotides, further preferably 25 - 50 nucleotides, most preferably 30 - 45 nucleotides.

The aptamer used in the present invention may be a conjugate selected from the group consisting of a conjugate of plural aptamers containing a nucleotide sequence represented by the above-mentioned formula (1) (aptamer (A)), a conjugate of plural aptamers containing a nucleotide sequence wherein 1 or several nucleotides are substituted, deleted, inserted or added in the nucleotide sequence represented by the above-mentioned formula (1) (aptamer (B)), and a conjugate of 1 or plural aptamers (A) and 1 or plural aptamers (B). These conjugates can also bind to FGF2.

Here, conjugation can be achieved by tandem binding. In the conjugation, a linker may be utilized. As the linker, nucleotide chains (e.g., 1 to about 20 nucleotides) and non-nucleotide chains (e.g., -(CH₂)ₙ- linker, -(CH₂CH₂O)ₙ- linker, hexaethylene glycol linker, TEG linker, peptide-containing linker, -S-S- bond-containing linker, -CONH- bond-containing linker, -OPO₃- bond-containing linker) can be mentioned. The plurality as mentioned in the above-described conjugate of a plurality thereof is not particularly limited, as long as it is two or more, and the plurality can be, for example, 2, 3 or 4.

Each nucleotide contained in the aptamer used in the present invention is the same or different and can be a nucleotide comprising a hydroxyl group at the 2'-position of ribose (e.g., ribose of pyrimidine nucleotide, ribose of purine nucleotide) (i.e., a natural nucleotide) or a nucleotide wherein hydroxyl group is substituted (modified) by any atom or group at the 2'-position of ribose (sometimes to be indicated as "modified nucleotide" in the present specification).

As examples of any such atom or group, a nucleotide substituted by a hydrogen atom, a fluorine atom or an -O-alkyl group (e.g., -O-Me group), an -O-acyl group (e.g., -O-CHO group), or an amino group (e.g., -NH₂ group) can be mentioned. In the aptamer used in the present invention, at least one kind (e.g., 1, 2, 3 or 4 kinds) of nucleotide can also be a modified nucleotide comprising a hydroxyl group, or the above-described any atom or group, for example, at least two kinds (e.g., 2, 3 or 4 kinds) of groups selected from the group consisting of a hydrogen atom, a fluorine atom, a hydroxyl group and a -O-Me group, at the 2'-position of ribose.

In the aptamer used in the present invention, all pyrimidine nucleotides may be nucleotides wherein the 2'-position of ribose is a fluorine atom, or may be the same or different and nucleotides wherein fluorine atom is unsubstituted, or substituted by any atom or group mentioned above, preferably an atom or group selected from the group consisting of a hydrogen atom, a hydroxyl group and a methoxy group. Particularly, when a production method using the DuraScribe^{™} T7 Transcription Kit (manufactured by Epicentre) is applied as a production method of the aptamer used in the present invention, an aptamer wherein the 2'-position of ribose of all pyrimidine nucleotides is fluorinated can be obtained. The aptamer wherein fluorine atom is substituted by other above-mentioned atom or group can be produced by the below-mentioned method.

In the aptamer used in the present invention, all purine nucleotides may be nucleotides wherein the 2'-position of ribose is a hydroxy group, or may be the same or different and nucleotides wherein hydroxy group is unsubstituted, or a nucleotide substituted by any atom or group mentioned above, preferably an atom or group selected from the group consisting of a hydrogen atom, a methoxy group and a fluorine atom at the 2'-position of ribose. The aptamer wherein a hydroxyl group is substituted by other above-mentioned atom or group can be produced by the below-mentioned method.

In the aptamer used in the present invention, all pyrimidine nucleotides may be nucleotides wherein the fluorine atom at the 2'-position of ribose is substituted by any of the aforementioned atoms or groups, for example, the same atoms or groups selected from the group consisting of a hydrogen atom, a hydroxy group and an -O-Me group.

In the aptamer used in the present invention, moreover, all purine nucleotides may be nucleotides wherein the hydroxy group at the 2'-position of ribose is substituted by any of the aforementioned atoms or groups, for example, the same atoms or groups selected from the group consisting of a hydrogen atom, a fluorine atom and an -O-Me group.

In a preferable embodiment, each pyrimidine nucleotide contained in the aptamer used in the present invention is a nucleotide containing a fluorine atom at the 2'-position of ribose, and each purine nucleotide is a nucleotide having a hydroxy group at the 2'-position of ribose. In another embodiment, the above-mentioned fluorine atom at the 2'-position of the ribose of each pyrimidine nucleotide is independently optionally substituted by an atom or group selected from the group consisting of a hydrogen atom, a hydroxy group and a methoxy group, and the above-mentioned hydroxy group at the 2'-position of the ribose of each purine nucleotide is optionally independently substituted by an atom or group selected from the group consisting of a hydrogen atom, a methoxy group and a fluorine atom.

In this description, the nucleotides constituting the aptamer are assumed to be RNAs (i.e., the sugar groups are assumed to be ribose) in describing how the sugar groups are modified in the nucleotides. However, this does not mean that DNA is exempted from the aptamer-constituting nucleotides, and a modification of RNA should read as a modification of DNA as appropriate. When the nucleotide constituting the aptamer is DNA, for example, replacement of the hydroxyl group at the 2'-position of ribose by X should read as a replacement of a hydrogen atom at the 2'-position of deoxyribose by X.

When uracil is substituted with thymine in the aptamer of the present invention, FGF2-binding activity, FGF2-FGF receptor binding inhibitory activity, stability, drug deliverability and stability in blood of the aptamer and the like can be increased.

In the aptamer used in the present invention, 1 or several, for example, 1 - 2, 1 - 3, 1 - 4, 1 - 5 nucleotides of phosphoric acid diester bond in the nucleotide may be modified or substituted by any substituent(s). For example, phosphoric acid diester bond may be substituted by a phosphorothioate bond, a phosphorodithioate bond, an alkylphosphonate bond, a phosphoramidate bond and the like. Here, for example, "nucleotide is substituted by a phosphorothioate bond" means that a phosphoric acid group at a binding site between adjacent nucleotides is sulfurated, that is, a phosphodiester bond is altered to a phosphorothioate bond.

In the aptamer used in the present invention, one or several, for example, 1 - 2, 1 - 3, 1 - 4, 1 - 5 nucleotides may be substituted by Bridged Nucleic Acid (BNA) or Locked Nucleic Acid (LNA) to stabilize aptamer and improve the activity thereof. As used herein, the "bridged nucleic acid" refers to one having a structure wherein the binding affinity to a complementary sequence is enhanced by restricting the degree of freedom of nucleic acid by intramolecular crosslinking, and acquire nuclease resistance. Examples thereof include, but are not limited to, 2',4'-BNA (Locked Nucleic Acid (LNA)), 2'-O,4'-C-ethylene-bridged Nucleic Acid (ENA) and the like.

The aptamer used in the present invention may be one wherein a sugar residue (e.g., ribose) of each nucleotide has been modified to increase the FGF2 binding activity, stability, drug deliverability and the like. As examples of the modification in a sugar residue, replacement of oxygen atom at the 2'-position, 3'-position and/or 4'-position of the sugar residue with another atom, and the like can be mentioned. As the kind of the modification, fluorination, O-alkylation (e.g., O-methylation, O-ethylation), O-arylation, S-alkylation (e.g., S-methylation, S-ethylation), S-arylation, and amination (e.g., -NH₂) can be mentioned. In addition, examples thereof include 4'-SRNA wherein the 4'-position oxygen is replaced with sulfur, LNA (Locked Nucleic Acid) wherein the 2'-position and the 4'-position are crosslinked via methylene, 3'-N-phosphoramidate nucleic acid wherein the 3'-position hydroxyl group is replaced with an amino group and the like. The aptamer used in the present invention is sometimes produced with a given modification of the oxygen atom at the 2'-position of ribose of pyrimidine nucleotide, due to the production method thereof. When a production method using, for example, DuraScribe^{™} T7 Transcription Kit (manufactured by Epicentre) is applied as a production method of the aptamer used in the present invention, an aptamer wherein the 2'-position of ribose of preferably all pyrimidine nucleotides is fluorinated is produced. Therefore, it is possible to produce various variations of aptamers having enhanced activity even though the base sequence is the same, by applying such alteration in the sugar residue to the obtained aptamer. From the above, the aptamer used in the present invention can be preferably an aptamer wherein a sugar residue of at least one nucleotide is modified. Such alterations in the sugar residue can be performed by a method known per se (see, for example, Sproat et al., (1991) Nucl. Acid. Res. 19, 733-738; Cotton et al., (1991) Nucl. Acid. Res. 19, 2629-2635; Hobbs et al., (1973) Biochemistry 12, 5138-5145). To be specific, an aptamer wherein the hydroxyl group at the 2'-position of ribose is substituted by an atom or group selected from the group consisting of a hydrogen atom, a hydroxyl group and a methoxy group can be produced by using, as a base, an aptamer wherein the hydroxyl group at the 2'-position of ribose of all pyrimidine nucleotides is substituted by a fluoro group.

The aptamer used in the present invention may also have a nucleic acid base (e.g., purine or pyrimidine) altered (e.g., chemical substitution) to enhance the FGF2 binding activity, prevention of multimerization, stability, drug deliverability and the like. As examples of such alterations, pyrimidine alteration at 5-position, purine alteration at 6- and/or 8-position(s), alteration with an extracyclic amine, substitution with 4-thiouridine, and substitution with 5-bromo or 5-iodo-uracil can be mentioned. The phosphate group contained in the aptamer used in the present invention may be altered to confer resistance to nuclease and hydrolysis. For example, the P(O)O group may be replaced with P(O)S (thioate), P(S)S (dithioate), P(O)N(R)R' (amidate), P(O)R, P(O)OR, CO or CH₂ (formacetal) or 3'-amine (-NH-CH₂-CH₂-) [wherein each unit of R or R' is independently H or a substituted or unsubstituted alkyl (e.g., methyl, ethyl)].

The linking group is, for example, -O-, -N- or -S-, and nucleotides can bind to an adjoining nucleotide via these linking groups.

The alterations may also include alterations such as capping at 3' and 5'.

An alteration can further be performed by adding to an end a polyethyleneglycol (PEG), amino acid, peptide, inverted dT, nucleic acid, nucleosides, Myristoyl, Lithocolic-oleyl, Docosanyl, Lauroyl, Stearoyl, Palmitoyl, Oleoyl, Linoleoyl, other lipids, steroids, cholesterol, caffeine, vitamins, dyes, fluorescent substances, anticancer agents, toxins, enzymes, radioactive substances, biotin and the like. For such alterations, see, for example, US Patents 5,660,985 and 5,756,703.

Particularly, when alteration is performed by terminus addition of PEG, the molecular weight of PEG is not particularly limited, and is preferably 1000 - 100000, more preferably 30000 - 90000. PEG may be linear or branched into two or more chains (multi-arm PEG). The terminus addition of PEG is useful for preventing the multimerization of the below-mentioned aptamer.

Such PEG is not particularly limited, and those of ordinary skill in the art can appropriately select and use commercially available or known PEG (e.g., http://www.peg-drug.com/peg_product/branched.html). Specific preferable examples of the PEG to be applied to the aptamer used in the present invention include 2-branched GS type PEG having a molecular weight of 40000 (SUNBRIGHT GL2-400GS manufactured by NOF CORPORATION), 2-branched TS type PEG having a molecular weight of 40000 (SUNBRIGHT GL2-400TS manufactured by NOF CORPORATION), 4-branched TS type PEG having a molecular weight of 40000 (SUNBRIGHT GL4-400TS manufactured by NOF CORPORATION), 2-branched TS type PEG having a molecular weight of 80000 (SUNBRIGHT GL2-800TS manufactured by NOF CORPORATION), 4-branched TS type PEG having a molecular weight of 80000 (SUNBRIGHT GL4-800TS manufactured by NOF CORPORATION) and the like.

In this case, in the aptamer used in the present invention, PEG may be directly added to the terminus. It is more preferable that a linker having a group bindable to PEG and the like be added to the terminus thereof, and PEG be added to the aptamer used in the present invention via the linker.

The linker for PEG and the aptamer used in the present invention is not particularly limited, and carbon chain number, functional group and the like can be appropriately selected according to the binding site, the kind of PEG and the like. Examples of such linker include a linker having an amino group. Specifically, when added to the 5' end, ssH Linker (SAFC) or DMS(O)MT-AMINO-MODIFIER (GLEN RESEARCH) can be mentioned, and when added to the 3' end, TFA Amino C-6 lcaa CPG (ChemGenes) and the like can be mentioned. When this linker is selected, for example, an active group of N-hydroxysuccinimide is added to PEG, and reacted with an amino group on the linker side, whereby the aptamer used in the present invention can be bound to PEG via the linker.

As PEG and linker, commercially available products can be preferably used. The reaction conditions and the like relating to the binding of PEG, a linker and the aptamer used in the present invention can be appropriately determined by those of ordinary skill in the art.

More preferred embodiments of the aptamer used in the present invention include aptamer ID1 containing the nucleotide sequence shown in SEQ ID NO: 3: 
GL2-400TS-C6-
G(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)C(M)-idT;
aptamer ID2 containing the nucleotide sequence shown in SEQ ID NO: 8: 
GL2-400TS-C6-
C(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)G(M)-idT;
aptamer ID3 containing the nucleotide sequence shown in SEQ ID NO: 9: 
GL2-400TS-C6-
C(M)C(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)G(M)G(M)-idT;
aptamer ID4 containing the nucleotide sequence shown in SEQ ID NO: 10: 
GL2-400TS-C6-
G(M)G(M)G(M)A(M)U(M)A(M)C(M)U(F)A(M)G(M)G(M)GC(M)G(M)U(M)U(F)A(
M)A(M)C(M)G(M)U(F)U(M)A(M)C(M)C(M)A(M)GU(F)GU(F)A(M)G(M)U(M)C(M
)C(M)C(M)-idT;
aptamer ID5 containing the nucleotide sequence shown in SEQ ID NO: 12: 
GL2-400TS-C6-
G(M)G(M)G(M)A(M)U(M)A(M)C(M)U(F)A(M)G(M)G(M)GC(M)A(M)U(M)U(F)U(
M)A(M)U(M)G(M)U(F)U(M)A(M)C(M)C(M)A(M)GU(F)GU(F)A(M)G(M)U(M)C(M
)C(M)C(M)-idT; and
aptamer ID6 containing the nucleotide sequence shown in SEQ ID NO: 3: 
idT-
G(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
 )C(M)C(M)-C6-GL2-400TS
In each of the above-mentioned formulas, the parentheses in each nucleotide indicate the modification of the 2'-position of ribose, F is a fluorine atom, and M is a methoxy group.

Irrespective of which of these aptamers is used, these aptamers can be stably present in the constitution of the aptamer preparation of the present invention, and thus are suitable as the active ingredient of the aptamer preparation of the present invention.

The aptamer used in the present invention may be a free form or a pharmaceutically acceptable salt thereof. Examples of such salt include metal salt, ammonium salt, organic amine addition salt, amino acid addition salt and the like. Examples of the metal salt include alkali metal salts such as sodium salt, potassium salt and the like, alkaline earth metal salts such as magnesium salt, calcium salt and the like, aluminum salt, zinc salt and the like. Examples of the ammonium salt include salts such as ammonium, tetramethylammonium and the like. Examples of the organic amine addition salt include salts such as trishydroxyaminomethane and the like. Examples of the amino acid addition salt include salts of lysine, arginine, histidine, tryptophan, ornithine and the like.

The concentration of the aptamer for FGF2 used in the present invention is not particularly limited, and the aptamer may be contained in any amount as long as the aptamer preparation of the present invention exerts the desired efficacy and effect. In the present specification, the "concentration of the aptamer" means the proportion (mg/mL) of the weight of the nucleic acid part linked by the 5'→3' phosphate bond constituting the aptamer molecule to the volume of the whole preparation. The concentration of the aptamer is, for example, 1 - 60 mg/mL.

When the concentration of the aptamer for FGF2 exceeds 20 mg/mL, a multimer of the aptamer tends to be easily produced even at the general storage temperature of 4 - 5°C. The aptamer for FGF2 when present as a monomer binds to FGF2, and inhibits the function of FGF2 and exerts the efficacy thereof. However, when it is multimerized, the binding to FGF2 is not observed in an assay using viacore. It is thus considered that the aptamer does not inhibit the function of FGF2 (see Example 4). When the concentration of the aptamer is low, the intermolecular distance is long and the tendency to multimerize is low. However, when the concentration of the aptamer exceeds 20 mg/mL, the aptamer is multimerized even at the general storage temperature of 4 - 5°C, as a result of which the binding activity to FGF2 tends to decrease as a whole (see Example 5). Therefore, the upper limit of the concentration of the FGF2 aptamer in the aptamer preparation of the present invention is desirably a concentration not exceeding 20 mg/mL. In addition, when the concentration of the aptamer in the aptamer preparation of the present invention is low, the effect as an injection may not be obtained. Therefore, while the lower limit of the concentration of the aptamer in the aptamer preparation of the present invention is not particularly limited as long as the effect as an injection can be obtained, it is desirably, for example, a concentration of not less than 1 mg/mL.

The dosage form of the aptamer preparation of the present invention is not particularly limited as long as it is an aqueous liquid. It is preferably an injection. Since the aptamer preparation of the present invention is an aqueous liquid, the aptamer for FGF2, which is the active ingredient, is dissolved in a solvent.

It is well known that aptamers generally require an inorganic salt, which is a strong electrolyte, to maintain the higher-order structure thereof. Macugen (registered trade mark), which is the only aptamer pharmaceutical product currently on the market, also contains sodium hydrogen phosphate, sodium dihydrogen phosphate, an isotonicity agent and a pH adjuster. Macugen in this state has been shown to be stable for 6 months in an accelerated test at 25±2°C and for 36 months in a long-term storage test at 5±3°C. From these, those of ordinary skill in the art should consider that an inorganic salt (electrolyte) is necessary for stable existence of the aptamer in an injection while maintaining the higher-order structure of the aptamer.

However, as shown in the below-mentioned Example, it was found that when PBS or physiological saline is used as the solvent, the above-mentioned aptamer for FGF2, which is the active ingredient in the present invention, tends to lose stability and form a multimer due to the influence of the inorganic salt which is a strong electrolyte and, as a result, loses the binding activity to FGF2. Therefore, the present inventors used water free of an inorganic salt (electrolyte) as a solvent and unexpectedly found that the multimerization of FGF2 aptamer was suppressed and the proportion of FGF2 aptamer present as a monomer increased (see Example 3).

Without wishing to be bound by theory, when an FGF2 aptamer is dissolved in water, the Tm value of the FGF2 aptamer cannot be measured as shown in Example 1, and the proton shift in NMR cannot be observed as shown in Example 2. Thus, the FGF2 aptamer dissolved in water is assumed to exist as a monomer in a state of being fully stretched. That is, it is thought that the higher-order structure of the aptamer, which is considered to be necessary for activity construction, is destroyed by using water free of inorganic salt as a solvent, denatured and exists in a single-stranded state, whereby deactivation (multimerization) can be prevented. Moreover, as shown in Examples 4 and 5, when the surface plasmon resonance (SPR) assay was performed by dissolving in a solution containing an electrolyte, the FGF2 aptamer maintained the binding activity to FGF2. Thus, it was demonstrated that when FGF2 aptamer dissolved in water is administered into the body, the higher-order structure required for aptamer activity is reconstructed by the action of the electrolyte present in the body fluid, and the activity can be exhibited.

As described above, It was revealed that a preparation formulation in which the higher-order structure of the FGF2 aptamer is intentionally destroyed to cause a fully-elongated state by dissolving the aptamer in a solvent free of an electrolyte, thereby also preventing multimerization during long-term storage and, after administration, the higher-order structure is re-constructed by exposure to a physiological salt concentration and the efficacy is exerted can eventually maintain the activity of the FGF2 aptamer for a long term. The present invention has been completed by verifying in detail the mode of existence of aptamer in such solution, and cannot be easily envisaged even by those skilled in the art.

From the above, the solvent used for the aptamer preparation of the present invention is preferably an aqueous solvent free of an inorganic salt (electrolyte), and water is particularly preferable.

As described above, since a solvent free of an inorganic salt (electrolyte) is used for the aptamer preparation of the present invention, the osmotic pressure is low and the preparation cannot be used as an injection as it is. Therefore, the aptamer preparation of the present invention is characterized in that it contains a non-electrolyte osmoregulator (osmolyte) for the purpose of adjusting the osmotic pressure ratio to the plasma osmotic pressure to 1 or more, preferably 1 - 3.

The osmoregulator used for the aptamer preparation of the present invention is mannitol.

The amount of the osmoregulator is not particularly limited, and those skilled in the art can appropriately change the amount of FGF2 aptamer in the preparation according to the kind (molecular weight) of the osmoregulator to be used and the target osmotic pressure. For example, when mannitol is used as the osmoregulator and the ratio of the osmotic pressure to the physiological saline is about 1, the blending ratio of the osmoregulator in the entire injection is 2 - 7.5%(w/v). More specifically, when the ratio of the osmotic pressure to the physiological saline is 1, the blending ratio of mannitol is 4.9% when the concentration of the above-mentioned FGF2 aptamer is 2 mg/ml, and the blending ratio of mannitol is 3.6% when the concentration of the above-mentioned FGF2 aptamer is 20 mg/ml.

The aptamer preparation of the present invention is preferably substantially free of an electrolyte other than the aptamer for FGF2 or a salt thereof to be the active ingredient. As used herein, "substantially free" means that a small amount of electrolyte may be contained as long as the FGF2 aptamer in the preparation can be stably stored for a long term. More preferably, the aptamer preparation of the present invention does not contains an electrolyte other than the aptamer for FGF2 or a salt thereof to be the active ingredient.

The aptamer preparation of the present invention may further contain a pharmaceutically acceptable additive where necessary. Examples of such additive include stabilizer, preservative, solubilizer, buffer, pH adjuster, soothing agent and the like, and well-known non-electrolyte pharmaceutical additives that are conventionally used as additives for injections can be preferably used.

The pH of the aptamer preparation of the present invention is not particularly limited. When the preparation is used as an injection, the pH is desirably near neutral, and can be appropriately selected within the range of, for example, 5 to 9, preferably 6 to 8. When an aptamer for FGF2 or a salt thereof, which is the active ingredient, is dissolved in water, the pH of the solution falls within the above-mentioned range. Therefore, it is not necessary to add an electrolyte pH adjuster or buffer to the aptamer preparation of the present invention.

The aptamer preparation of the present invention may contain other active ingredients as long as they do not adversely affect the activity and stability of the FGF2 aptamer. As such active ingredient, VGEF inhibitors such as McGen (registered trade mark), Lucentis (registered trade mark), Eylea (registered trade mark), Avastin (registered trade mark) and the like as therapeutic drugs for diseases accompanied by angiogenesis and the like, anti-inflammatory agents such as steroids and the like, human growth hormone preparations such as Norditropin (registered trade mark) and Genotropin (registered trade mark) as therapeutic agents for bone diseases and the like, and analgesic/sedative agents such as morphine may be contained. Examples thereof include pharmaceutical compounds for treating or preventing diseases accompanied by angiogenesis such as age-related macular degeneration and the like, bone or cartilage diseases such as osteoporosis, rheumatoid arthritis, osteoarthritis, bone fracture and the like, and pain.

The aptamer preparation of the present invention having the above-mentioned constituent is stable at 4°C for not less than 3 months. As used herein, "stable" means that the proportion of a monomer aptamer in the preparation after storage of the preparation enclosed in a glass bottle at 4°C is not less than 70%, as mentioned above. Preferably, in the aptamer preparation of the present invention, the proportion of a monomer aptamer in the preparation after storage at 4°C for 3 months is not less than 80%. In addition, the aptamer preparation of the present invention is stable even when exposed to white fluorescent lighting or near-ultraviolet fluorescent lighting.

Therefore, the aptamer preparation of the present invention can be stored stably for a long term in the form of an aqueous liquid as is, preferably in a dosage form of an injection such as a pre-filled syringe, cartridge or the like, by refrigerating at not more than 5°C, and is extremely easy to handle.

The aptamer preparation of the present invention can be preferably used as, for example, a medicament for the treating or preventing diseases accompanied by angiogenesis such as age-related macular degeneration and the like, bone or cartilage diseases such as osteoporosis, rheumatoid arthritis, osteoarthritis, bone fracture and the like.

The aptamer preparation of the present invention can be administered parenterally (e.g., intravenous administration, subcutaneous administration, intramuscular administration, topical administration, intraperitoneal administration, intranasal administration, pulmonary administration, instillation administration and the like). The dosage of the aptamer preparation of the present invention varies depending on the kind and activity of FGF2 aptamer, seriousness of disease, animal species being the subject of administration, drug tolerability of the subject of administration, body weight, age and the like, and the usual dosage, based on the amount of active ingredient (oligonucleotide site of aptamer) per day for an adult, can be about 0.0001 to about 100 mg/kg, for example, about 0.0001 to about 10 mg/kg, preferably about 0.005 to about 1 mg/kg.

### [Example]

The present invention is explained in more detail in the following by referring to Examples; however, the present invention is not limited by the Examples.

### Example 1 (Structural analysis of FGF2 aptamer in solvent : determination of Tm value)

The structure of the aptamer shown in aptamer ID1 is shown below. Capital letter shows RNA, small letter shows DNA, and idT shows inverted dT. The parenthesis in each nucleotide shows modification at the 2'-position thereof, F shows a fluorine atom, and M shows an O-methyl group. C6 shows - (CH₂)₆-linker. PEG40TS2 is 2-branched TS type polyethylene glycol having a molecular weight of 40000 (SUNBRIGHT GL2-400TS manufactured by NOF CORPORATION).
aptamer ID1:

GL2-400TS-C6-
G(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)C(M)-idT

The aptamer represented by aptamer ID1 was dissolved in water to a concentration of 0.1 mg/mL and in PBS or physiological saline to a concentration of 0.06 mg/mL. The obtained solution was heated at 95°C for 5 min, cooled to room temperature and filled in a quartz glass cuvette. The Tm value was determined by measuring UV absorption with a spectrophotometer while changing the temperature from 20°C to 90°C.

As a result, the Tm value was 60.1°C in PBS, and 69.6°C in physiological saline. On the other hand any particular Tm value was not observed in water. From this, it was assumed that the aptamer represented by aptamer ID1 forms a higher-order structure by intermolecular interaction in PBS or physiological saline, which can generally be a solvent for injections. In addition, it was assumed that the aptamer represented by aptamer ID1 does not form a base pair between molecules or within molecule, and does not form a higher-order structure in water free of electrolyte.

### Example 2 (Structural analysis of FGF2 aptamer in deuterium oxide: measurement of NMR spectrum)

A 20 mg aptamer represented by aptamer ID1 was filled in a glass vial and dissolved in about 1 mL of deuterium oxide to prepare a measurement sample. It was measured using a Bruker Avance 600 MHz NMR spectrometer. As a result, an iminoproton signal derived from base pairs formation, which should be observed in a magnetic field lower than 8 ppm, was not observed.

Therefrom, it was assumed that the aptamer represented by aptamer ID1 does not form a base pair between molecules or within molecule, and does not form a higher-order structure in water free of electrolyte.

### Example 3 (Structural analysis of FGF2 aptamer in solution containing electrolyte: SEC-MALS measurement)

The aptamer represented by aptamer ID1 was dissolved in physiological saline to a concentration of 20 mg/mL, and incubated at 37°C for 2 weeks to artificially prepare an FGF2 aptamer preparation that forms a higher-order structure. The preparation and a sample obtained by freezing and storing immediately after preparation to minimize the formation of higher-order structure were each diluted with physiological saline to 0.2 mg/mL, and subjected to analysis.

Monomers and multimers were separated by size-exclusion chromatography, and respective molecular weights were measured with MALS (Multi Angle Light Scattering) detector manufactured by Wyatt Technology. The size-exclusion chromatography was performed by ACQUITY UPLC manufactured by Waters and using a BEH200 SEC column.

As a result, the molecular weight of the peak that seemed to be a monomer was measured to be about 64000, and the molecular weight of the peak that seemed to be a higher-order structure composed of multimers was measured to be about 122000. From the results, it was found that the higher-order structure formed by the aptamer represented by aptamer ID1 in water containing an electrolyte is a dimer.

### Example 4 (Correlation between monomer content percentage and binding activity of aptamer)

The aptamer represented by aptamer ID1 was dissolved in PBS, physiological saline or 3.3% mannitol aqueous solution to a concentration of 20 mg/mL or 2 mg/mL to prepare FGF2 aptamer preparations that show various monomer content percentages under the storage conditions shown in Table 1. The monomer content of each of the prepared FGF2 aptamer preparations was determined by size-exclusion chromatography. The results thereof are also shown in Table 1.

The binding activity of each of the prepared FGF2 aptamer preparations to FGF2 protein was measured by surface plasmon resonance (SPR) using Biacore T200 manufactured by GE. As the sensor chip, CM4 that reacts with an amino group was used. Human FGF2 was dissolved in immobilization solution (10 mM sodium acetate, pH 6) at 10 µg/ml. For the reaction of an amino group on the protein side and a carboxyl group on the chip side, ethyl-3-carbodiimide hydrochloride and N-hydroxysuccinimide were used. After the reaction, blocking by ethanolamine was performed. The immobilized amount of FGF2 was set to 1000 RU. An aptamer for analyte was prepared to 5 µM. As a running buffer, 295 mM sodium chloride, 5.4 mM potassium chloride, 0.8 mM magnesium chloride, 1.8 mM calcium chloride, 20 mM Tris, 0.05% Tween20, pH 7.6) were used, and 2M sodium chloride was used as a regeneration solution. FGF2 was immobilized on a flow cell FC2 or FC4, and the results of FC1 or FC3 were subtracted to give a final sensorgram. The activity was evaluated as a relative value to the FGF2 aptamer preparation standard having an extremely low multimer content and prepared when in use using a solution free of an electrolyte.

Table 1 shows the preparation method, monomer content percentage, and the measurement results of the binding activity to FGF2 protein of respective FGF2 aptamer preparations. From the results, it was clarified that the monomer content of the FGF2 aptamer preparation and the binding activity to the FGF2 protein are correlated.

**[Table 1]**

| Monomer content percentage and binding activity to FGF2 protein FGF2 aptamer preparation | | | |
|---|---|---|---|
| FGF2 aptamer preparation | preparation method (storage conditions) | monomer content percentage (%) | binding activity (%) |
| 2 mg/mL 3.3% mannitol preparation | preparation on demand | 98.7 | 98.1 |
| 20 mg/mL 3.3% mannitol preparation | preparation on demand | 91.3 | 91.6 |
| 20 mg/mL 3.3% mannitol preparation | 25°C 1 week | 80.5 | 75.7 |
| 20 mg/mL 3.3% mannitol preparation | 25°C 2 weeks | 71.5 | 65.2 |
| 20 mg/mL 3.3% mannitol preparation | 25°C 1 month | 63.4 | 57.2 |
| 20 mg/mL 3.3% mannitol preparation | 25°C 3 months | 50.2 | 50.2 |
| 2 mg/mL physiological saline | 37°C 2 days | 56.9 | 56.2 |
| 2 mg/mL PBS | 37°C 2 days | 57.2 | 60.6 |
| 20 mg/mL physiological saline | 37°C 2 days | 12.1 | 18.3 |
| 20 mg/mL PBS | 37°C 2 days | 11.3 | 13.1 |

### Example 5 (Stability test of FGF2 aptamer preparation)

The aptamer represented by aptamer ID1 was dissolved in 3.3% or 3.6% or 4.9% mannitol solution to a concentration of 20 mg/mL or 2 mg/mL, stored for 3 months under various temperature conditions shown in Table 2, and the monomer content percentage and binding activity were measured by size-exclusion chromatography and the SPR method. The results are shown in Table 2.

From the results, it was clarified that an FGF2 aptamer preparation prepared using a mannitol aqueous solution free of electrolyte is stable.

**[Table 2]**

| Stability test results of FGF2 aptamer preparation | | | | |
|---|---|---|---|---|
| preparation concentration (mg/mL) | solution | storage temperature | monomer content percentage (%) | binding activity (%) |
| 2 | 3.3% mannitol | -20°C | 98.4 | 102.0 |
| 2 | 3.3% mannitol | 5°C | 99.1 | 102.7 |
| 20 | 3.3% mannitol | -20°C | 89.2 | 91.0 |
| 20 | 3.3% mannitol | 5°C | 88.5 | 90.8 |
| 2 | 4.9% mannitol | -20°C | 98.3 | 101.0 |
| 2 | 4.9% mannitol | 5°C | 98.0 | 103.1 |
| 20 | 3.6% mannitol | -20°C | 88.8 | 84.1 |
| 20 | 3.6% mannitol | 5°C | 88.0 | 81.8 |

### Example 6 (Results in other FGF2 aptamer preparations)

The aptamers represented by aptamer ID2 - 6 are dissolved in water (mannitol aqueous solution), physiological saline or PBS to appropriate concentrations to prepare FGF2 aptamer preparations. The respectively obtained FGF2 aptamer preparations are stored under various temperature conditions and storage temperature for several months, and the monomer content percentage and binding activity are measured by size-exclusion chromatography and the SPR method.
aptamer ID2: 
GL2-400TS-C6-
C(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)G(M)-idT;
aptamer ID3: GL2-400TS-C6- 
idT-

G(M)G(M)G(M)A(M)U(M)A(M)C(M)U(F)A(M)G(M)G(M)GC(M)A(M)U(M)U(F)A(

M)A(M)U(M)G(M)U(F)U(M)A(M)C(M)C(M)A(M)GU(F)GU(F)A(M)G(M)U(M)C(M

C(M)C(M)G(M)A(M)U(M)A(M)C(M)U(F)A(M)G(M)G(M)GC(M)A(M)U(M)U(F)A(

M)A(M)U(M)G(M)U(F)U(M)A(M)C(M)C(M)A(M)GU(F)GU(F)A(M)G(M)U(M)C(M

)G(M)G(M)-idT;
aptamer ID4: 
GL2-400TS-C6-
G(M)G(M)G(M)A(M)U(M)A(M)C(M)U(F)A(M)G(M)G(M)GC(M)G(M)U(M)U(F)A(
M)A(M)C(M)G(M)U(F)U(M)A(M)C(M)C(M)A(M)GU(F)GU(F)A(M)G(M)U(M)C(M
)C(M)C(M)idT-;
aptamer ID5: 
GL2-400TS-C6-
G(M)G(M)G(M)A(M)U(M)A(M)C(M)U(F)A(M)G(M)G(M)GC(M)A(M)U(M)U(F)U(
M)A(M)U(M)G(M)U(F)U(M)A(M)C(M)C(M)A(M)GU(F)GU(F)A(M)G(M)U(M)C(M
)C(M)C(M)idT-; and
aptamer ID6:

idT-
G(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)C(M)-C6-GL2-400TS

From these results, it is clarified that other FGF2 aptamer preparations prepared using a mannitol aqueous solution free of electrolyte are also stable.

### Comparative Example 1 (Stability test of FGF2 aptamer preparation)

The aptamer represented by aptamer ID1 was dissolved in physiological saline or PBS to a concentration of 20 mg/mL to prepare FGF2 aptamer preparations. The respectively obtained FGF2 aptamer preparations were stored for 3 months under various temperature conditions shown in Table 3, and the monomer content percentage and binding activity were measured by size-exclusion chromatography. The results are shown in Table 3. From the results, it was clarified that FGF2 aptamer preparations prepared using physiological saline and PBS containing electrolyte are unstable.

**[Table 3]**

| Stability test results of FGF2 aptamer preparations prepared using solution containing electrolyte | | | |
|---|---|---|---|
| preparation concentration (mg/mL) | solution | storage temperature | monomer content percentage (%) |
| 20 | physiological saline | -20°C | 65.4 |
| 20 | physiological saline | 5°C | 44.5 |
| 20 | 3.3% mannitol | -20°C | 68.2 |
| 20 | 3.3% mannitol | 5°C | 36.8 |

### [Industrial Applicability]

The aptamer preparation of the present invention can stably store an aptamer for FGF2 or a salt thereof for a long term in the form of an aqueous liquid such as injection or the like by refrigerating. Therefore, it is extremely useful in that it can provide a preparation superior in handling as a therapeutic or prophylactic agent for diseases for which inhibition of FGF2 can exhibit efficacy (e.g., diseases accompanied by angiogenesis such as age-related macular degeneration and the like, bone or cartilage diseases such as osteoporosis, rheumatoid arthritis, osteoarthritis, bone fracture and the like, pain).

## Claims

1. An aqueous liquid comprising an aptamer or a salt thereof that binds to FGF2, and a non-electrolytic osmoregulator, wherein the aptamer or a salt thereof is stable for a long term such that the proportion of a monomer aptamer after storage of the preparation enclosed in a glass bottle at 4°C for 3 months is not less than 70%, and wherein the osmoregulator is mannitol.

2. The aqueous liquid according to claim 1, wherein the liquid is substantially free of an electrolyte other than the aptamer or a salt thereof.

3. The aqueous liquid according to claim 1, wherein the aptamer comprises a nucleotide sequence represented by the following formula (1) (wherein uracil is optionally thymine):
N¹GGAN²ACUAGGGCN³UUAAN⁴GUN⁵ACCAGUGUN⁶ (1)
(wherein N¹ and N⁶ are each independently any 0 to several bases, and
N², N³, N⁴ and N⁵ are independently any one base),
and is the following (a) or (b):
(a) an aptamer wherein, in the nucleotides contained in the aptamer,
(i) the 2'-position of the ribose of each pyrimidine nucleotide is a fluorine atom,
(ii) the 2'-position of the ribose of each purine nucleotide is a hydroxy group;
(b) an aptamer wherein, in the nucleotides contained in the aptamer,
(i) the 2'-position of the ribose of each pyrimidine nucleotide is independently unsubstituted, or substituted by an atom or group selected from the group consisting of a hydrogen atom, a methoxy group and a fluorine atom,
(ii) the 2'-position of the ribose of each purine nucleotide is independently unsubstituted, or substituted by an atom or group selected from the group consisting of a hydrogen atom, a methoxy group and a fluorine atom.

4. The aqueous liquid according to claim 1 or 2, wherein the aptamer comprises a nucleotide sequence represented by the following formula (3):
N¹GGAUACUAGGGCAUUAAUGUUACCAGUGUAGUCN⁶² (3)
(wherein N¹ and N⁶² are each independently any 0 to several bases).

5. The aqueous liquid according to claim 1 or 2, wherein the aptamer comprises a nucleotide sequence shown in SEQ ID NO: 3, 8, 9, 10 or 12.

6. The aqueous liquid according to any one of claims 1 to 5, wherein the aptamer has a concentration of 1 - 60 mg/mL.

7. The aqueous liquid according to any one of claims 1 to 6, wherein the osmoregulator is blended at a proportion of 2 - 7.5%(w/v) of the whole aqueous liquid.

8. The aqueous liquid according to any one of claims 1 to 7, wherein the mannitol is contained at 1 - 50 mg per 1 mg of the aptamer.

9. The aqueous liquid according to any one of claims 1 to 8, wherein the liquid is stored at not more than 5°C.

10. The aqueous liquid according to any one of claims 1 to 9, wherein a proportion of a monomer aptamer is not less than 80% after storage at 4°C for 3 months.

11. The aqueous liquid according to any one of claims 1 to 10, wherein the liquid is for injection.

12. The aqueous liquid according to any one of claims 1 to 11 for use in the prophylaxis or treatment of a disease accompanied by angiogenesis, bone or cartilage disease or pain.

## Patentansprüche

1. Wässrige Flüssigkeit, umfassend ein Aptamer oder ein Salz davon, das an FGF2 bindet, und einen nicht-elektrolytischen Osmoregulator, wobei das Aptamer oder ein Salz davon für eine langen Zeitraum stabil ist, so dass der Anteil eines monomeren Aptamers nach Lagerung der in einer Glasflasche bei 4°C für 3 Monate eingeschlossenen Zubereitung nicht weniger als 70% beträgt, und wobei der Osmoregulator Mannitol ist.

2. Wässrige Flüssigkeit nach Anspruch 1, wobei die Flüssigkeit im Wesentlichen frei von einem anderen Elektrolyten als dem Aptamer oder einem Salz davon ist.

3. Wässrige Flüssigkeit nach Anspruch 1, wobei das Aptamer eine Nukleotidsequenz umfasst, die durch die folgende Formel (1) dargestellt ist (wobei Uracil gegebenenfalls Thymin ist):
N¹GGAN²ACUAGGGCN³UUAAN⁴GUN⁵ACCAGUGUN⁶ (1)
(wobei N¹ und N⁶ jeweils unabhängig beliebige 0 bis mehrere Basen sind, und N², N³, N⁴ und N⁵ unabhängig eine beliebige Base sind),
und die folgende (a) oder (b) ist:
(a) ein Aptamer, wobei in den im Aptamer enthaltenen Nukleotiden,
(i) die 2'-Position der Ribose jedes Pyrimidin-Nukleotids ein Fluoratom ist,
(ii) die 2'-Position der Ribose jedes Purinnukleotids eine Hydroxygruppe ist;
(b) ein Aptamer, wobei in den im Aptamer enthaltenen Nukleotiden,
(i) die 2'-Position der Ribose jedes Pyrimidinnukleotids unabhängig unsubstituiert, oder durch ein Atom oder eine Gruppe ausgewählt aus der Gruppe bestehend aus einem Wasserstoffatom, einer Methoxygruppe und einem Fluoratom, substituiert ist,
(ii) die 2'-Position der Ribose jedes Purinnukleotids unabhängig unsubstituiert, oder durch ein Atom oder eine Gruppe ausgewählt aus der Gruppe bestehend aus einem Wasserstoffatom, einer Methoxygruppe und einem Fluoratom, substituiert ist.

4. Wässrige Flüssigkeit nach Anspruch 1 oder 2, wobei das Aptamer eine Nukleotidsequenz umfasst, die durch die folgende Formel (3) dargestellt ist:
N¹GGAUACUAGGGCAUUAAUGUUACCAGUGUAGUCN⁶² (3)
(wobei N¹ und N⁶² jeweils unabhängig beliebige 0 bis mehrere Basen sind).

5. Wässrige Flüssigkeit nach Anspruch 1 oder 2, wobei das Aptamer eine in SEQ ID NO: 3, 8, 9, 10 oder 12 dargestellte Nukleotidsequenz umfasst.

6. Wässrige Flüssigkeit nach einem der Ansprüche 1 bis 5, wobei das Aptamer eine Konzentration von 1 - 60 mg/ml aufweist.

7. Wässrige Flüssigkeit nach einem der Ansprüche 1 bis 6, wobei der Osmoregulator mit einem Anteil von 2 - 7,5% (w/v) der gesamten wässrigen Flüssigkeit gemischt ist.

8. Wässrige Flüssigkeit nach einem der Ansprüche 1 bis 7, wobei das Mannitol in einer Menge von 1 - 50 mg pro 1 mg des Aptamers enthalten ist.

9. Wässrige Flüssigkeit nach einem der Ansprüche 1 bis 8, wobei die Flüssigkeit bei nicht mehr als 5°C gelagert ist.

10. Wässrige Flüssigkeit nach einem der Ansprüche 1 bis 9, wobei ein Anteil eines monomeren Aptamers nach Lagerung bei 4°C für 3 Monate nicht weniger als 80% beträgt.

11. Wässrige Flüssigkeit nach einem der Ansprüche 1 bis 10, wobei die Flüssigkeit zur Injektion ist.

12. Wässrige Flüssigkeit nach einem der Ansprüche 1 bis 11 zur Verwendung bei der Prophylaxe oder Behandlung einer Krankheit, die von Angiogenese, Knochen- oder Knorpelerkrankungen oder Schmerzen begleitet ist.

## Revendications

1. Liquide aqueux comprenant un aptamère ou un sel de celui-ci qui se lie à FGF2, et un osmorégulateur non électrolytique, dans lequel l'aptamère ou un sel de celui-ci est stable sur une longue période de façon que la proportion d'un aptamère monomère après stockage de la préparation enfermée dans une bouteille en verre à 4°C pendant 3 mois ne soit pas inférieure à 70 %, et dans lequel l'osmorégulateur est le mannitol.

2. Liquide aqueux selon la revendication 1, lequel liquide est pratiquement exempt d'un électrolyte autre que l'aptamère ou un sel de celui-ci.

3. Liquide aqueux selon la revendication 1, dans lequel l'aptamère comprend une séquence de nucléotides représentée par la formule (1) suivante (dans laquelle l'uracile est éventuellement la thymine) :
N¹GGAN²ACUAGGGCN³UUAAN⁴GUN⁵AACAGUGUN⁶ (1)
(dans laquelle chacun de N¹ et N⁶ représente indépendamment l'une quelconque parmi 0 à plusieurs bases, et
N², N³, N⁴ et N⁵ sont indépendamment n'importe quelle base),
et est l'un de (a) et (b) suivants :
(a) un aptamère dans lequel, dans les nucléotides contenus dans l'aptamère,
(i) la position 2' du ribose de chaque nucléotide pyrimidine est un atome de fluor,
(ii) la position 2' du ribose de chaque nucléotide purine est un groupe hydroxy ;
(b) un aptamère dans lequel, dans les nucléotides contenus dans l'aptamère,
(i) la position 2' du ribose de chaque nucléotide pyrimidine est indépendamment non substituée ou substituée par un atome ou groupe choisi dans l'ensemble constitué par un atome d'hydrogène, un groupe méthoxy et un atome de fluor,
(ii) la position 2' du ribose de chaque nucléotide purine est indépendamment non substituée ou substituée par un atome ou groupe choisi dans l'ensemble constitué par un atome d'hydrogène, un groupe méthoxy et un atome de fluor.

4. Liquide aqueux selon la revendication 1 ou 2, dans lequel l'aptamère comprend une séquence de nucléotides représentée par la formule (3) suivante :
N¹GGAUACUAGGGCAUUAAUGUUACCAGUGUAGUCN⁶² (3)
(dans laquelle chacun de N¹ et N⁶² représente indépendamment l'une quelconque parmi 0 à plusieurs bases).

5. Liquide aqueux selon la revendication 1 ou 2, dans lequel l'aptamère comprend une séquence de nucléotides indiquée dans la SEQ ID NO : 3, 8, 9, 10 ou 12.

6. Liquide aqueux selon l'une quelconque des revendications 1 à 5, dans lequel l'aptamère a une concentration de 1 à 60 mg/mL.

7. Liquide aqueux selon l'une quelconque des revendications 1 à 6, dans lequel l'osmorégulateur est mélangé en une proportion de 2 à 7,5 % (p/v) du liquide aqueux total.

8. Liquide aqueux selon l'une quelconque des revendications 1 à 7, dans lequel le mannitol est contenu à raison de 1 à 50 mg par mg de l'aptamère.

9. Liquide aqueux selon l'une quelconque des revendications 1 à 8, lequel liquide est stocké à au plus 5°C.

10. Liquide aqueux selon l'une quelconque des revendications 1 à 9, dans lequel la proportion d'aptamère monomère n'est pas inférieure à 80 % après 3 mois de stockage à 4°C.

11. Liquide aqueux selon l'une quelconque des revendications 1 à 10, lequel liquide est pour injection.

12. Liquide aqueux selon l'une quelconque des revendications 1 à 11, pour une utilisation dans la prophylaxie ou le traitement d'une maladie s'accompagnant d'une angiogenèse, d'une maladie des os ou du cartilage, ou d'une douleur.
